Europäisches Patentamt

European Patent Office

Office européen des brevets

(19)

(11) Numéro de publication: **0 106 756**
**A1**

(12)

# DEMANDE DE BREVET EUROPEEN

(21) Numéro de dépôt: **83401949.9**

(22) Date de dépôt: **05.10.83**

(51) Int. Cl.³: **A 61 B 5/04**

(30) Priorité: **05.10.82 FR 8216706**
**04.02.83 FR 8301796**
**13.09.83 FR 8314517**
**13.09.83 FR 8314518**

(43) Date de publication de la demande:
**25.04.84 Bulletin 84/17**

(84) Etats contractants désignés:
**AT BE CH DE GB IT LI LU NL SE**

(71) Demandeur: **Ascher, Gilles**
**20bis, Boulevard du Général Leclerc**
**F-92200 Neuilly(FR)**

(71) Demandeur: **Coustenoble, Jean-Pierre**
**11, rue Charcot**
**F-92800 Puteaux(FR)**

(72) Inventeur: **Liber, François Odon**
**8, rue de Soubise**
**F-59140 Dunkerque(FR)**

(72) Inventeur: **Ascher, Gilles**
**20bis, Bld du Général Leclerc**
**F-92200 Neuilly(FR)**

(72) Inventeur: **Coustenoble, Jean-Pierre**
**11, rue Charcot**
**F-92800 Puteaux(FR)**

(74) Mandataire: **Rodhain, Claude et al,**
**Cabinet Claude RODHAIN 30, rue La Boétie**
**F-75008 Paris(FR)**

(54) Appareil portatif d'enregistrement d'électrocardiogrammes.

(57) L'invention concerne un appareil portatif destiné à l'enregistrement d'électrocardiogrammes qui comprend un boitier (1) de faibles dimensions comportant sur au moins une de ses faces extérieures (9), deux électrodes d'enregistrement d'électrocardiogrammes (7,8) destinées à recevoir chacune une partie d'un des deux bras et un dispositif électronique de traitement disposé dans ledit boitier et comprenant des circuits de numérisation, de mémorisation, et de conversion sous forme analogique du signal électrique détecté.

L'appareil est porté en permanence par le patient qui procède à un enregistrement d'électrocardiogrammes dès qu'il ressent un symptôme.

L'électrocardiogramme ainsi obtenu est ensuite mis sous forme numérique et stocké dans une mémoire, et il pourra être converti sous forme analogique et recueilli par le médecin cardiologue lors de la consultation.

## FIG.1

EP 0 106 756 A1

"Appareil portatif d'enregistrement d'électrocardiogrammes".

L'invention concerne les appareils d'enregistrement d'électrocardiogrammes dont l'examen permet aux médecins cardiologues d'établir un diagnostic.

Parmi les patients qui viennent consulter un cardiologue, il en est de nombreux qui présentent des symptômes (douleurs, palpitations, vertiges...) à fréquence faible, par exemple une douleur par semaine, que lesexamens traditionnels ne permettent pas de mettre en évidence.

En effet, si l'utilité de l'electrocardiogramme de consultation n'est plus à démontrer dans le diagnostic de cardiopathies typiques, il s'avère par contre bien souvent inefficace pour préciser l'origine d'un symptôme isolé qu'il est impossible de reproduire au moment de l'examen. On a donc prévu des examens destinés à mettre en évidence de tels symptômes à fréquence faible.

C'est ainsi que l'on peut soumettre le patient à une épreuve d'effort, dont le but est de placer le coeur dans des conditions de travail telles que l'on puisse diagnostiquer certaines anomalies. Mais cet examen n'offre pas toutes les garanties souhaitables, en particulier lorsque le symptôme est lié à un aspect précis de la vie du patient (activité professionnelle, stress particulier...) et inexistant au moment de l'examen. En outre, le caractère relativement lourd de cet examen peut être disproportionné par rapport à la gravité réelle des maux dont souffre le patient.

On connait également la méthode de l'enregistrement ambulatoire de l'électrocardiogramme par la méthode de Holter, qui permet de surveiller le patient pendant l'exercice de ses activités quotidiennes ; mais l'intérêt de cet examen est limité par la durée maximale de l'enregistrement, qui est généralement de 24 heures, avec possibilité d'extension jusqu'à 48 heures pour certains appareillages.

Enfin, des essais ontété faits à l'aide d'appareils qui sont portés par le patient pendant une période pouvant aller jusqu'à une semaine, et qui sont munis d'un microprocesseur programmé pour enregistrer les seuls évènements pathologiques ; mais les limites actuelles de l'électronique d'analyse font que la fiabilité d'un tel enregistreur est loin d'être satisfaisante, de plus la gêne évidente présentée par la nécessité de garder plusieurs jours des électrodes collées sur la poitrine et reliées par un câble à un enregistreur assez volumineux, constitue un inconvénient quasi rédhibitoire.

Faute de pouvoir faire un diagnostic précis, le médecin cardiologue est donc bien souvent conduit à prescrire des traitements d'essais avec le cortège d'aléas que cela comporte.

La présente invention a pour but de remédier à ces inconvénients et de permettre, même pour des symptômes à fréquence très faible, d'enregistrer un électrocardiogramme au moment où se produit ce symptôme.

L'appareil selon l'invention comprend un boîtier de faibles dimensions comportant sur au moins une de ses faces extérieures, deux électrodes d'enregistrement d'électrocardiogrammes destinées à recevoir chacune une partie d'un des deux bras et un dispositif électronique de traitement disposé dans ledit boîtier et comprenant des circuits de numérisation, de mémorisation et de conversion sous forme analogique du signal électrique détecté.

L'appareil est porté en permanence par le patient qui procède à un enregistrement d'électrocardiogrammes dès qu'il ressent un symptôme.

L'électrocardiogramme ainsi obtenu est ensuite mis sous forme numérique et stocké dans une mémoire et il pourra être converti sous forme analogique et recuelli par le médecin cardiologue lors de la consultation.

On voit donc que l'appareil selon l'invention permet à n'importe quel patient d'enregistrer en toute circonstance un électrocardiogramme, lorsqu'il ressent un symptôme.

Selon un premier mode de réalisation de l'invention, le boîtier est un boîtier plat de format de poche et les deux électrodes sont disposées sur une même face du boîtier et reçoivent chacune un ou plusieurs doigts d'une main.

Selon un deuxième mode de réalisation de l'invention, le boîtier est un boîtier plat du type boîtier de montre-bracelet maintenu sur le bras au moyen d'un bracelet, la première électrode est disposée sur la face du boîtier qui est en contact avec le bras et la deuxième électrode est disposée sur la face opposée extérieure du boîier et destinée à recevoir une partie de l'autre bras telle qu'un doigt.

L'invention permet donc d'obtenir un dispositif de faible encombrement qui est porté en permanence par le patient et qui est donc immédiatement disponible ; une des électrodes est constamment en contact avec le patient et il suffit de porter le doigt sur la deuxième électrode extérieure pour pouvoir procéder à un enregistrement d'électrocardiogrammes.

D'autres caractéristiques et avantages de l'invention ressortiront de la description qui suit, faite à titre illustratif et nullement limitatif en se référant aux dessins ci-annexés sur lesquels :

- la fig.1 est une vue en perspective d'un mode de réalisation de l'appareil selon l'invention ;

- la fig.2 est une vue de dessus correspondant à la fig.1 ;

- la fig.3 est un schéma-bloc du dispositif électronique de traitement du signal électrique détecté ;

- la fig.4 est une vue de détail d'une variante de réalisation de l'invention ;

- la fig.5 représente en perspective un autre mode de réalisation de l'appareil selon l'invention ;

- les fig.6 et 7 représentent des détails de réalisation des moyens de retenue ;

- la fig.8 représente une variante de réalisation des movens de retenue ;

- la fig.9 représente en perspective un autre mode de réalisation de l'appareil portatif selon l'invention ;

- la fig.10 est une vue de dessus du boîtier d'un mode de réalisation de l'invention ;

- la fig.11 est une vue de gauche correspondant à la vue de la fig.1 ;

- la fig.12 est le schéma-bloc d'un mode de réalisation du dispositif électronique de traitement, et

- la fig.13 représente le schéma-bloc d'un autre mode de réalisation de l'invention.

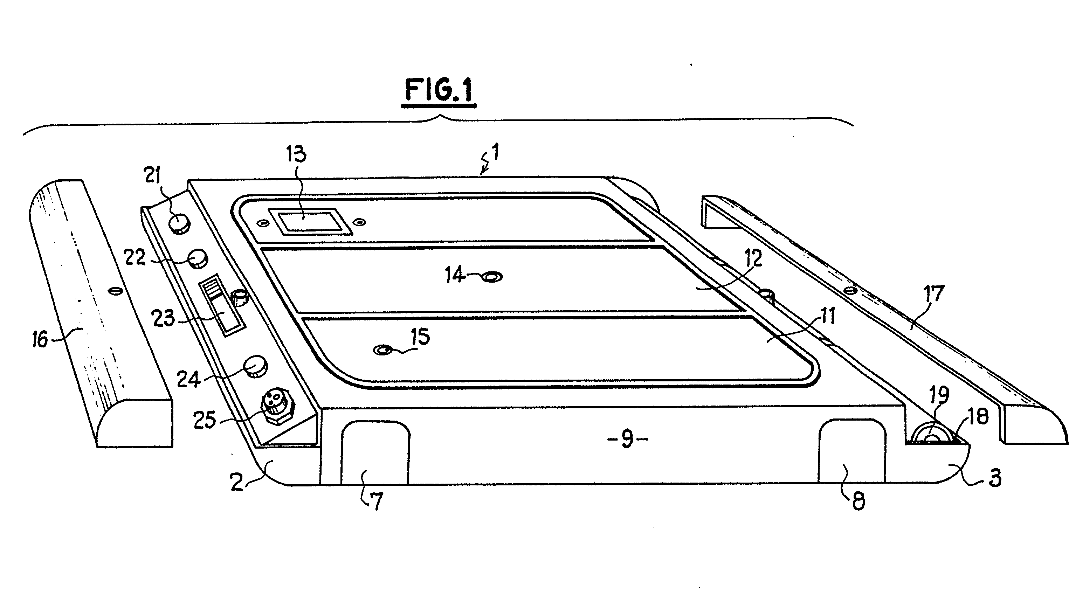

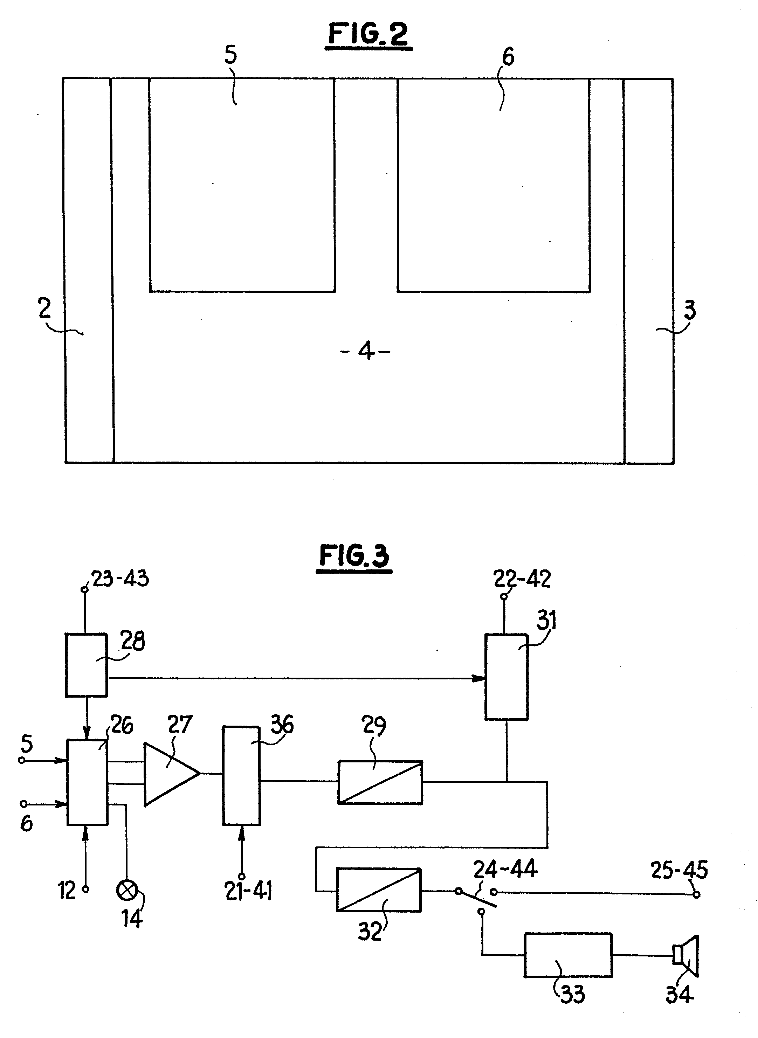

L'appareil représenté aux fig.1 et 2 est essentiellement constitué d'un boîtier 1 de forme générale plate et de faible dimension; les bords 2 et 3 de ce boîtier sont arrondis de manière à faciliter son insertion dans une poche ou dans un sac, ce qui permet de l'avoir toujours à portée de la main. Les dimensions extérieures de ce boîtier peuvent par exemple être de 155 x 94 x 20 mm.

La face inférieure 4 du boîtier 1 comporte deux électrodes 5 et 6 d'électrocardiographe dont la surface est importante de manière à recevoir les quatre doigts d'une main opposés au pouce. A titre d'exemple, les dimensions de chacune de ces électrodes peuvent être de 40 x 50 mm. Elles sont réalisées en tout matériau approprié pour l'enregistrement de l'électrocardiogramme, tel que par exemple des alliages présentant une conductibilité élevée par rapport à la peau ; on peut par exemple utiliser l'alliage connu sous le nom commercial d'"arcap".

Chaque électrode 5, respectivement 6 est reliée à une électrode 7, respectivement 8, qui est disposée sur une face latérale 9 du boîtier, et qui, dans l'exemple représenté constitue un prolongement de l'électrode 6, respectivement 6. Ces électrodes supplémentaires 7 et 8 sont de surface plus faible et elles sont disposées à une distance maximale l'une de l'autre de manière à réaliser un enregistrement précordial, comme cela sera expliqué plus loin.

Sur sa face supérieure 11, l'appareil selon l'invention comporte un dispositif de la détection de la présence des pouces ; dans l'exemple représenté, ce dispositif est constitué par une touche 12 de grande surface à effleurement. Sur cette face supérieure 11 se trouvent également une montre digitale 13 et un voyant de fonctionnement 14. Enfin, un orifice 15, disposé également sur cette face supérieure 11, est destiné à la transmission par voie téléphonique de l'électrocardiogramme enregistré.

Les deux bords 2 et 3 du boîtier 1 comportent chacun un couvercle 16 respectivement 17 ; le couvercle 17 recouvre un logement 18 recevant les piles électriques d'alimentation 19 et le couvercle 16 découvre des éléments de commande qui ne sont normalement accessibles qu'au médecin. Un bouton 21 commande le réglage de la bande passante de l'appareil, un bouton 22 commande les opérations de lecture et d'effacement de la mémoire, un curseur 23 sert à programmer le cycle et la durée des enregistrement et un bouton 24 sert au choix de la transmission, soit par voie téléphonique, soit directement sur un électrocardiographe chez le cardiologue, l'appareil selon l'invention étant relié à l'électrocardiographe par la connexion 25.

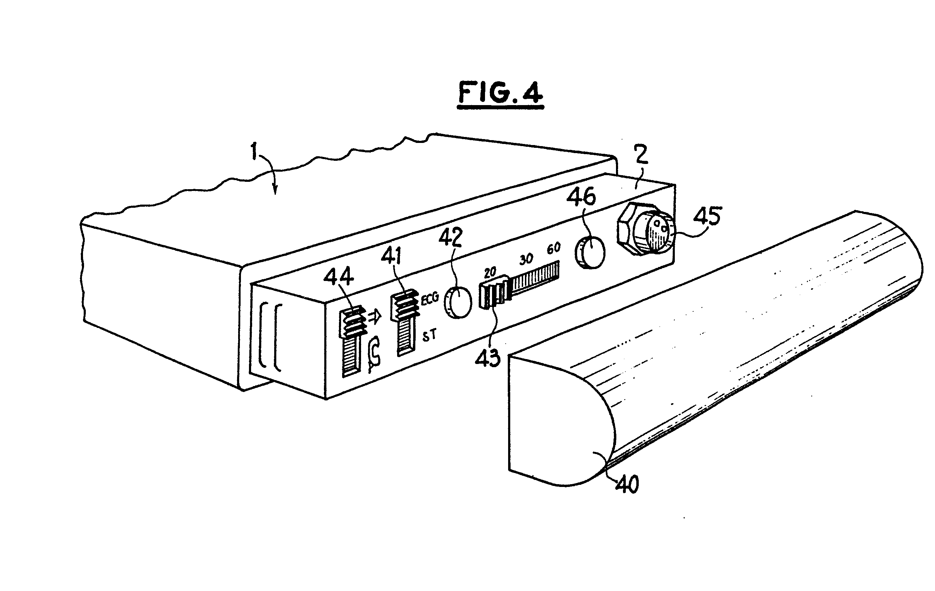

La fig.4 représente une variante de réalisation du tableau de commande de l'appareil selon l'invention. Le couvercle 40 est de forme semi-cylindrique, et les boutons de commande 41 et 44, qui correspondent aux boutons 21 et 24 sont du type à curseur, ce qui permet de visualiser le mode de fonctionnement de l'appareil. On retrouve ainsi le curseur 44 de choix de la transmission, directe ou téléphonique, le curseur 41 du choix de la bande passante, le curseur 43 du choix de la séquence et de la durée, le bouton 42 de commande de lecture et d'effacement de la mémoire, et la connexion 45. De plus, un bouton 46 est prévu pour le test des piles d'alimentation de l'appareil.

Le fonctionnement de l'appareil selon l'invention sera expliqué en se référant à la fig.3 qui est un schéma-bloc du dispositif électronique qui est logé dans le boîtier 1 et qui sert à la commande de l'enregistrement et au traitement du signal électrique détecté.

Les signaux provenant des électrodes d'enregistrement 5 et 6 sont envoyés par l'intermédiaire d'un dispositif de commande d'enregistrement 26 sur un circuit d'amplification et de mise en forme du signal 27, constitué de préférence par un amplificateur différentiel à haute impédance d'entrée. L'enregistrement d'un électrocardiogramme est déclenché par le signal provenant de la touche 12 de détection de la présence du pouce sur le boîtier ; la fréquence et la durée des enregistrements sont commandées par un circuit 28 lui-même commandé par le bouton de commande de programmation 23.

Le signal fourni par l'amplificateur opérationnel 27 est transformé en un signal numérique au moyen d'un convertisseur analogique-numérique 29, et le signal numérique obtenu est mémorisé dans une mémoire 31 commandée par le circuit 28. Les signaux numériques représentatifs de l'électrocardiogramme qui a été enregistré sont stockés dans la mémoire 31 jusqu'à ce que le médecin ou le patient vide cette mémoire ; cette opération de lecture de l'électrocardiogramme peut se faire de deux manières, le choix étant fait par le bouton-commutateur 24 ou le curseur 44. Dans les conditions normales, le patient se présente chez son médecin cardiologue qui procède lui-même à la lecture de l'électrocardiogramme sur l'électrocardiographe de son cabinet. A cet effet, le dispositif électronique selon l'invention comporte un deuxième convertisseur 32 numérique-analogique, qui transforme les signaux mémorisés dans la mémoire 31 en signaux analogiques directement lisibles par un électrocardiographe et fournis sur la prise 25. L'enregistrement dans la mémoire 31 est commandé de telle manière que le contenu de cette mémoire ne peut être "écrasé" par un nouvel enregistrement, c'est-à-dire que la mémoire 31 stocke le ou les premiers électrocardiogrammes détectés. La mémoire 31 ne peut être effacée que par le médecin en agissant sur le bouton 22 ou 42 qui commande d'une part, la lecture de la mémoire, et d'autre part, l'effacement de cette mémoire lorsque le médecin a bien transféré l'électrocardiogramme. En d'autres termes, le patient ne peut pas détruire les premiers enregistrements, car s'il cherche à en effectuer d'autres et si la mémoire 31 est pleine, l'appareil ne se déclenche pas, ce qui lui sera indiqué par l'absence d'allumage du voyant 14 qui lui indique alors qu'il a épuisé les possibilités d'enregistrement.

Si le patient désire transmettre plus rapidement l'électrocardiogramme qu'il vient d'enregistrer à son médecin, il peut le faire par voie téléphonique ; à cet effet, le dispositif électronique comprend un circuit d'interface téléphonique 33 relié au commutateur 24 ; avantageusement, le circuit d'interface est constitué par un modulateur à modulation de fréquence dont la sortie est envoyée sur un transducteur téléphonique 34 disposé en regard de l'orifice 15. L'utilisation d'une modulation de fréquence permet d'affranchir l'électrocardiogramme transmis des parasites de l'onde porteuse. Le médecin doit alors disposer dans son cabinet d'un récepteur approprié effectuant la démodulation pour restituer l'électrocardiogramme sous forme analogique.

Dans un mode de réalisation de l'invention, la fréquence de l'onde porteuse est de 2000 Hz et le facteur de modulation est de 100 hz/mV.

On voit que l'invention fournit un appareil qui permet à tout patient de pouvoir enregistrer un ou plusieurs électrocardiogrammes au moment où se produisent des symptômes cardiaques, même de fréquence très lente. Il lui suffit de prendre l'appareil, de poser les mains sur les électrodes 5 et 6, les pouces sur les touches 12 et l'enregistrement se fait avec la fréquence et la durée programmées par le médecin, grâce au curseur 23. Le voyant 14 indique au patient que l'enregistrement est en cours, et lui permet de relacher l'appareil dès que cet enregistrement est terminé.

Le médecin quant à lui, n'a qu'à ouvrir le couvercle 16, brancher son électrocardiogramme sur la prise 25 et il obtient aussitôt, sans problème d'adaptation, l'électrocardiogramme de son patient.

Selon une autre caractéristique importante de l'invention, l'appareil comporte les deux électrodes supplémentaires 7 et 8 qui permettent d'effectuer un enregistrement précordial de l'électrocardiogramme chez le médecin, en appliquant tout simplement l'appareil sur la poitrine du patient.

Ceci permet d'éviter tout parasite d'origine musculaire provenant des bras et d'élargir la bande passante de manière à obtenir un enreigstrement plus précis, en particulier au niveau des fréquences très basses.

Selon une variante de réalisation de l'invention, cet enregistrement précordial se fait entre la poitrine et la main droite du patient, et, dans ce cas, les électrodes 7 et 8 sont disposées sur deux faces latérales opposées du boîtier et leurs dimensions sont plus importantes.

Cette disposition correspond à la dérivation CM5 de l'électrocardiogramme qui permet de mettre en évidence les anomalies du segment S T correspondant aux troubles de la repolarisation, c'est-à-dire d'état des coronaires ; cela permet de déceler entre autre, un infarctus en formation.

Pour obtenir un électrocardiogramme plus prévis dans le cabinet par enregistrement précordial, le dispositif électronique comprend avantageusement un filtre 36 commandé par le bouton 31, ce qui permet de faire un enregistrement précordial avec une bande passante plus élevée, et donc d'obtenir un électrocardiogramme plus précis. En effet, pour l'enregistrement de l'électrocardiogramme par les doigts, la bande passante est réduite de manière à éliminer les courants parasites tels que les courants musculaires par exemple. Ainsi, dans un mode de réalisation de l'invention, la bande passante est de 0,5 à 20 Hz pour l'enregistrement de l'électrocardiogramme par les doigts, et de 0,05 à 40 Hz pour l'enregistrement précordial. Dans ce mode de réalisation, la taille de la mémoire 31 est de 18 K octets et la fréquence d'échantillonnage du convertisseur analogique-numérique 29 est de 100 Hz.

La présence d'une montre 13 sur le boîtier 1 permet au patient de noter l'heure à laquelle s'est produit le symptôme et à laquelle a été fait l'enregistrement, ce qui peut être une information importante pour le médecin. Selon une variante de réalisation de l'invention, cette heure précise d'enregistrement est enregistrée en même temps que l'électrocardiogramme et indiquée ainsi de manière automatique au médecin lors de la restitution de l'électrocardiogramme. Ceci peut, par exemple, être obtenu dans un appareil selon l'invention, dans lequel le dispositif électronique est essentiellement constitué par un microprocesseur commandant les différentes opérations.

Les séquences et durées d'enregistrement pouvant être choisies par le médecin, peuvent par exemple être, soit un enregistrement de 180 secondes, trois enregistrements de 60 secondes, six enregistrements de 30 secondes, et neuf enregistrements de 20 secondes.

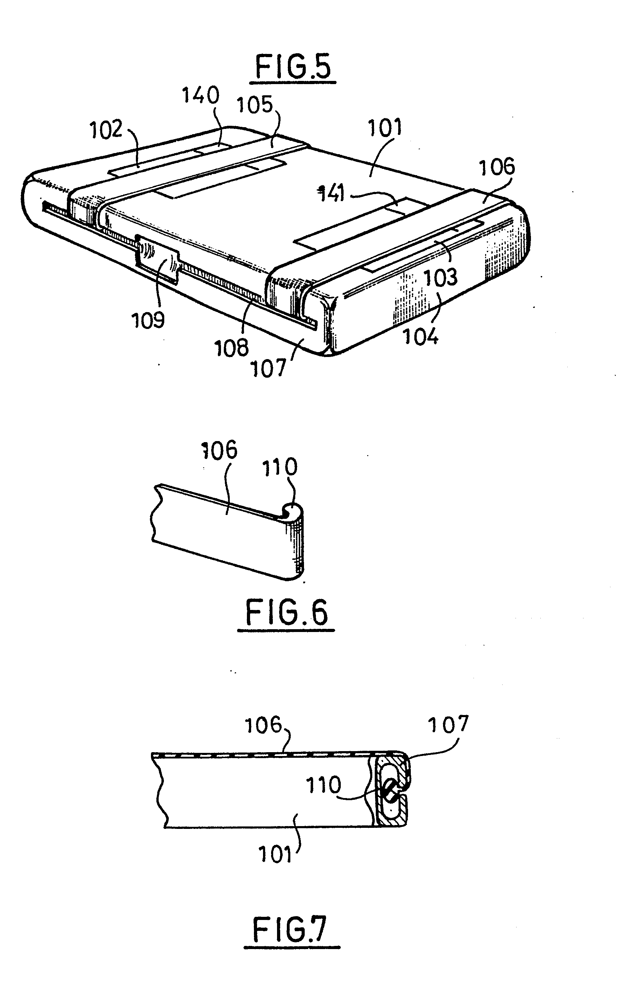

On voit sur la fig.5 un appareil destiné à l'enregistrement d'un électrocardiogramme qui est essentiellement constitué d'un boîtier 101 de forme plate et de format réduit, pouvant être mis dans une poche ; sur la face supérieure de ce boîtier sont prévues deux électrodes de recueil d'électrocardiogrammes 102 et 103 qui sont destinées à recevoir une partie de chaque main du patient, en l'occurence les quatre doigts faisant face au pouce. Pour ce faire, le boîtier 101 est pris entre les deux mains, la paume d'une main s'appuyant sur le petit côté 104 du boîtier et la paume de l'autre main sur le petit côté opposé du boîtier 101, les pouces venant se placer sur la face inférieure du boîtier.

Conformément à l'invention, on prévoit des moyens de retenue de la partie de la main venant en contact avec les électrodes 102 et 103 , ces moyens de retenue appliquent la partie considérée de la main sur lesdites électrodes avec une pression constante. Le patient n'étant pas obligé d'appuyer ses mains sur le boîtier puisqu'elles y sont maintenues, n'aura donc pas tendance à se crisper. Dans le mode de réalisation représenté à la fig.1, ces moyens de retenue sont élastiques et constitués par deux bandes élastiques 105 et 106 disposées sensiblement en regard des électrodes et s'étendant sur toute la largeur du boîtier 101. Ces bandes de maintien 105 et 106 sont fixées sur les deux faces latérales du boîtier 101 sur lesquelles la main ne vient pas en contact lors de l'enregistrement de l'électrocardiogramme.

A cet effet, ces faces latérales (dont une seule référencée 107 est visible sur la fig.5) présentent une fente longitudinale en forme de queue d'aronde 108 dont la section est représentée à la fig.6. Cette fente longitudinale comporte une échancrure 109 disposée de préférence au centre et dont la largeur correspond approximativement au fond de la fente 108.

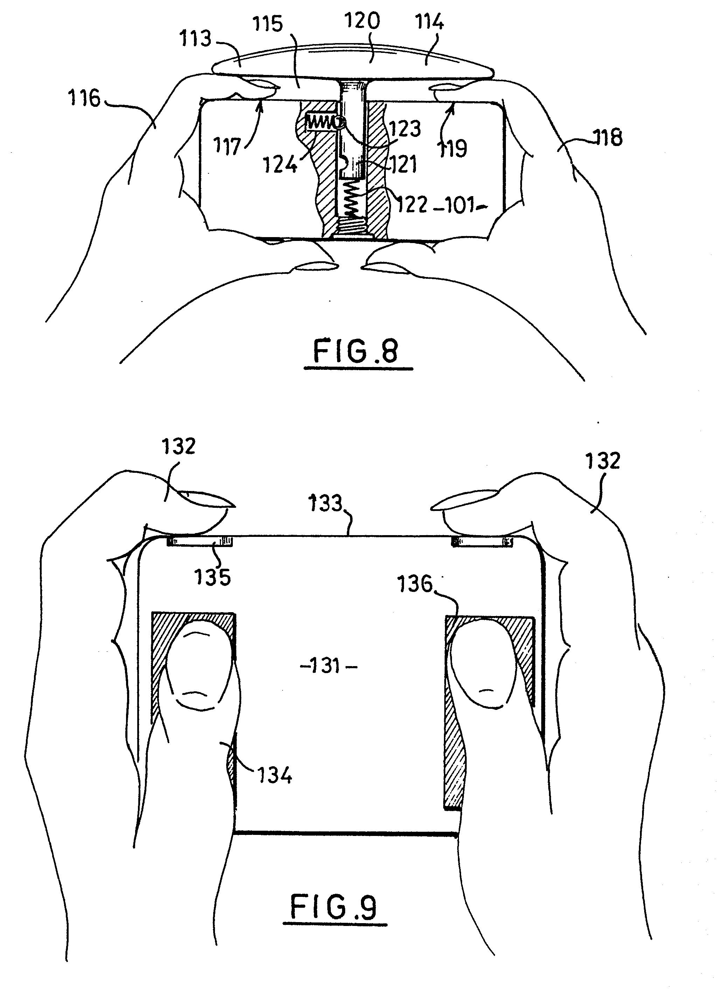

Par ailleurs, les extrémités des bandes 105 et 106 présentent une section élargie 110 (voir fig.7) dont la section correspond à la largeur de l'échancrure 109 ; cette section élargie peut être par exemple obtenue en enroulant l'extrémité des bandes 105 et 106 sur elle-même. On voit que l'extrémité 110 des bandes 105 et 106 peut être engagée dans l'échancrure 109 puis glissée dans la fente 108 pour amener les bandes 105 et 106 au regard des électrodes 102 et 103.

La fig.8 montre une variante de réalisation des moyens de retenue ; ces derniers sont constitués par deux pattes 113 et 114 solidaires d'une des faces latérales du boîtier 101 ; ces pattes 113 et 114 sont relativement élastiques et forment une sorte de ressort qui délimite avec la face latérale du boîtier 101 un logement 115 pour un doigt. Dans ce cas, on insère les doigts sous les pattes 113 et 114 qui se resserrent sur les doigts et les maintiennent en place contre un élément de mesure ou de déclenchement. Dans l'exemple représenté, le doigt 116, par exemple l'index, est appliqué contre un élément de déclenchement de l'enregistrement de l'électrocardiogramme 117 et l'autre doigt 118 est appliqué contre un capteur 119 destiné à recueillir les courbes systolique et diastolique de la pression sanguine.

Selon un mode de réalisation qui est représenté à la fig.8, les deux pattes 113 et 114 constituent la barre horizontale d'une pièce indépendante 120 en forme de T dont la barre verticale 121 est montée à coulissement dans le boîtier 101. Cette pièce en forme de T 120 est rappelée vers le boîtier 101 au moyen d'un dispositif de rappel schématisé par le ressort 122. Ce dispositif de retenue constitué par la pièce 120 comporte un dispositif de blocage schématisé sur la fig.8 par une bille 123 soumise à l'action d'un ressort 124 et coopérant avec une encoche de la partie verticale 121 de la pièce 120. Avantageusement, le dispositif de blocage qui est destiné à maintenir la pièce de retenue 120 en position écartée du boitier 101 de manière à permettre le logement facile des doigts 116 et 118, comporte un élément de déblocage de manière à ramener la pièce de retenue 120 en appui sur les doigts du patient en vue de l'enregistrement de l'électrocardiogramme. Cet élément de déblocage peut être de tout type connu et agit par exemple sur la bille 123 ; il est actionné par une autre partie de la main du patient. Avantageusement, cet élément de déblocage peut être couplé au dispositif de déclenchement de l'enregistrement.

La fig.9 représente une variante de réalisation de l'invention selon laquelle le boitier 131 présente la forme générale et les dimensions approximatives d'un paquet de cigarettes. Dans ce mode de réalisation, l'enregistrement de l'électrocardiogramme est effectué sur les index, tels que 132, qui viennent s'appuyer sur une électrode d'électrocardiogramme 135 située sur un des grands côtés 133 du boitier 131 et y sont maintenus par un mpyen de retenue tel que décrit plus haut. Dans ce mode de réalisation, au moins un des pouces tels que 134 vient en contact avec un dispositif 136 servant au déclenchement de l'enregistrement et au déblocage du dispositif de verrouillage dans le cas de moyens de retenue tels que décrits à la fig.8. L'enregistrement des courbes de la pression sanguine s'effectue alors sur l'un des index.

Du fait que les électrodes d'enregistrement de l'électrocardiogramme sont disposées sur un grand côté du boîtier 133, il existe entre ces électrodes une distance suffisamment grande qui permet d'utiliser l'appareil selon l'invention en enregistrement précordial de manière à affiner encore les caractéristiques de la courbe recueillie. Si l'on veut effectuer un enregistrement précordial, on utilise alors des moyens de retenue élastiques tels que ceux décrits aux fig. 5 à 7, de manière à pouvoir dégager les électrodes en vue de cet enregistrement précordial.

Lorsque l'appareil selon l'invention comprend un dispositif d'enregistrement des courbes systolique et diastolique de la pression sanguine, il est nécessaire que le médecin dispose en son cabinet d'un décodeur spécialisé qui permette par exemple d'obtenir un enregistrement graphique de ces courbes.

On peut voir sur la fig.5 un autre dispositif qui permet d'améliorer encore la qualité des électrocardiogrammes recueillis et enregistrés. Selon cette variante, pour éviter des interférences (myogrammes) dues à des artéfacts musculaires, les électrodes de recueil de l'électrocardiogramme 102 et 103 ne s'étendent que sur seulement environ les deux tiers de la cavité recevant les doigts ou toute autre partie de la main ; l'autre partie des électrodes 102 et 103 repérée 140 respectivement 141, est isolée du reste de l'électrode, par exemple par une paroi, et constitue une électrode de masse qui est reliée au circuit électronique de traitement des électrocardiogrammes recueillis.

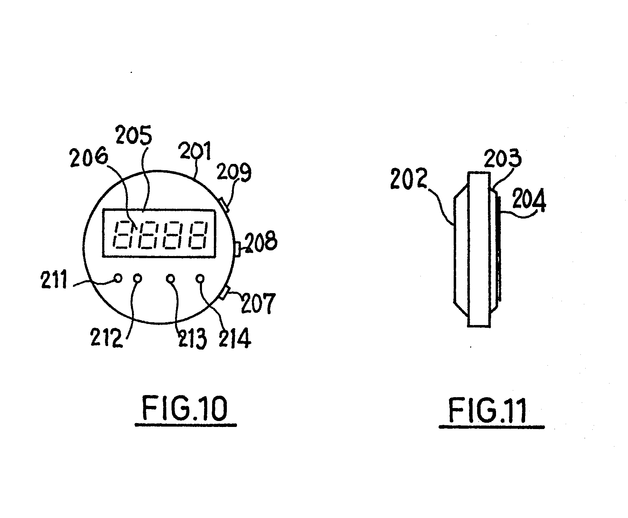

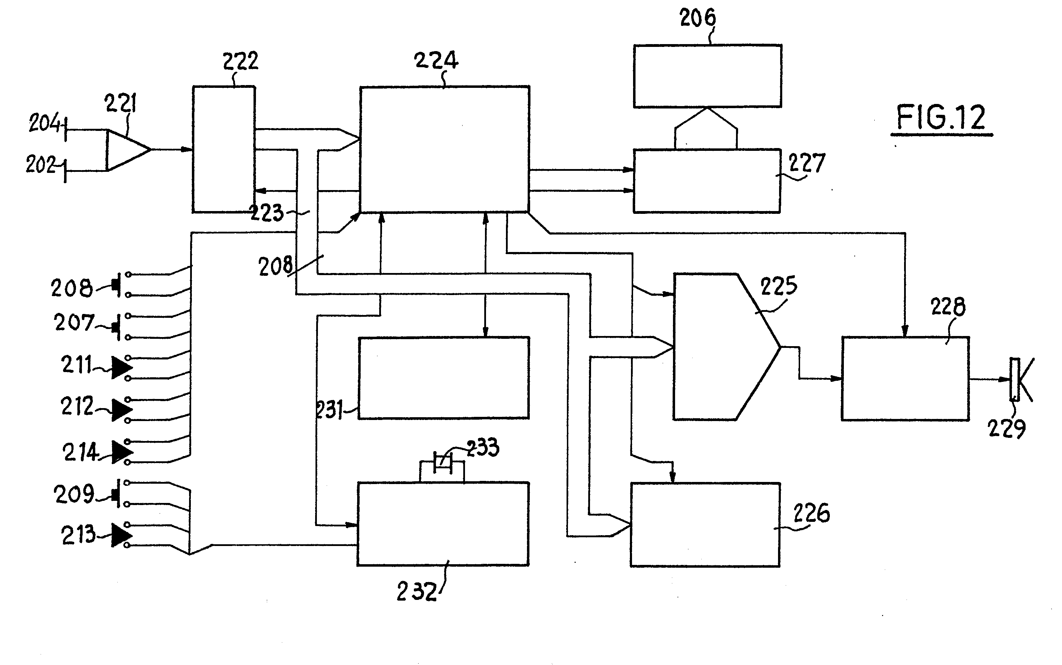

Sur les fig.10 et 11, on a représenté un mode de réalisation du boîtier constituant l'appareil selon l'invention. Ce boîtier 201 est de faibles dimensions, il est plat et sa forme correspond par exemple à celle d'un boîtier de montre-bracelet. Ce boîtier est porté par le patient comme une montre au moyen d'un bracelet (non représenté) qui le maintient sur un des poignets.

Le fond métallique 202 du boîtier 201 constitue une des électrodes qui est en contact permanent avec l'un des bras du patient. L'autre électrode est disposée sur la face extérieure ou couvercle 203 du boîtier ; elle est par exemple constituée par une métallisation 204 réalisée sur un couvercle transparent 203. Comme on peut le voir sur la fig.10, cette métallisation laisse libre une fenêtre d'affichage 205 qui permet de voir les indications fournies par un dispositif d'affichage 206 qui est avantageusement constitué par des cristaux liquides de manière à limiter la consommation de l'appareil. Le couvercle transparent 203 est réalisé en un matériau qui permet une tenue durable de la métallisation constituant l'électrode 204 ; on peut par exemple, utiliser du saphir synthétique.

Avantageusement, comme on le verra plus loin, l'appareil selon l'invention est couplé à une montre à affichage numérique qui utilise le dispositif d'affichage 206.

Le boîtier comporte également trois boutons 207,208 et 209, et quatre poussoirs 211, 212,213 et 214 dont l'utilisation sera expliquée plus loin.

Le boîtier 201 comprend, outre le dispositif d'affichage 206 un dispositif électronique de traitement du signal enregistré sur les bornes 202 et 204. Ce dispositif électronique est réalisé sous forme de circuits intégrés, ce qui permet de le loger facilement dans le boîtier. La fig.12 représente un exemple de réalisation d'un tel dispositif électronique.

On retrouve sur la fig.12 les trois boutons 207, 208 et 209 et les autres poussoirs 211 à 214. Le signal fourni par les électrodes 202 et 204 est tout d'abord envoyé sur un amplificateur différentiel à haute impédance d'entrée 221 dont les signaux de sortie sont convertis sous forme numérique par un convertisseur analogique-numérique 222. Ce convertisseur 222 est par exemple un convertisseur à huit bits et travaille à un rythme de 100 échantillons par seconde.

Les signaux à huit bits obtenus sont envoyés, par l'intermédiaire d'un bus 223 à une unité logique de commande et de traitement 224, à un convertisseur numérique-analogique 225 et à une mémoire 226 qui est avantageusement constituée par une mémoire vive (RAM) de 2 kilo-octets. Le dispositif d'affichage à cristaux liquides 206 est commandé par une unité de commande d'affichage 227.

Conformément à l'invention, le dispositif électronique de traitement comprend un dispositif de transmission acoustique essentiellement constitué par un oscillateur commandé par la tension (VCO) 223 qui reçoit les signaux analogiques fournis par le convertisseur 225 et dont le signal de sortie assure l'excitation d'un transducteur acoustique 229 constitué par exemple par un piézo-transducteur.

Le dispositif de traitement électronique comporte également un circuit 231 qui sert au calcul du rythme cardiaque pendant l'enregistrement de l'électrocardiogramme, et avantageusement, comme indiqué plus haut, une horloge 232 en temps réel utilisant le dispositif d'affichage 206.

L'unité logique de commande et de traitement 224 commande le fonctionnement des différents composants précités. Ce fonctionnement est le suivant. Lors de l'enregistrement d'un électrocardiogramme, le signal fourni par les électrodes est amplifié puis numérisé par le convertisseur 222 ; le signal obtenu est mémorisé dans la mémoire 226 et il est par ailleurs envoyé à l'unité logique de commande et de traitement qui, en coopération avec le circuit 231 qui est destiné plus particulièrement à détecter la partie QRS de l'électrocardiogramme enregistré, calcule de manière continue pour chaque complexe d'électrocardiogrammes le rythme cardiaque correspondant. La valeur du rythme ainsi obtenue est envoyée à l'unité de commande 227 qui en commande l'affichage sur les cristaux liquides 206. Cet affichage est commandé par le bouton 207 du boîtier.

L'unité logique de commande et de traitement 224 peut également effectuer une détection de seuil de tachycardie et de brachycardie, le dépassement d'un de ces seuils entrainant le déclenchement d'une alarme acoustique par l'intermédiaire du transducteur 229. Avantageusement, les seuils de tachycardie et de brachycardie peuvent être réglés au moyen des poussoirs 212 et 211 respectivement.

L'appareil peut également comporter un bouton 208 destiné à la commande de l'enregistrement par le patient, ce bouton étant actionné par un deuxième doigt du patient.

La lecture de l'enregistrement mémorisé dans la mémoire 226, enregistrement qui correspond par exemple à une durée de 20 secondes, est commandée par un poussoir 214. Lorsque l'on commande de cette manière la lecture, l'unité logique de commande et de traitement 224 commande la lecture de la mémoire 226 dont le contenu est envoyé par le bus 223 dans le convertisseur 225 qui restitue une courbe analogique d'électrocardiogrammes qui commande l'oscillateur variable 228 dont le signal de sortie module l'onde acoustique émise par le transducteur 229. Le médecin dispose dans son cabinet d'un appareil récepteur de ce signal acoustique qui comporte un dispositif de démodulation qui peut par exemple être constitué par un décodeur à interpolation linéaire ; cette opération s'effectuant sans aucun branchement entre les deux appareils. Cette transmission peut également être réalisée par l'intermédiaire d'une ligne téléphonique sans aucune adaptation.

Comme indiqué plus haut, il est particulièrement avantageux que l'appareil selon l'invention soit couplé avec une montre 222 à affichage numérique commandée par un quartz 233 ; cette montre comporte de manière connue, un bouton 209 de commande de l'affichage de l'heure et un poussoir 213 de mise à l'heure.

Pour réduire la consommation de l'appareil, lorsque l'appareil est au repos, c'est-à-dire qu'il fonctionne en montre uniquement, seuls les circuits logiques centraux sont alimentés, à savoir l'horloge 232, l'unité logique 224, le circuit 231, le circuit de commande de l'interphase 227 et la mémoire 226.

Dans le cas où l'appareil selon l'invention est couplé avec une montre, il est possible de prévoir un couvercle formant électrode extérieure et découvrant, lorsqu'il est ouvert, le cadran de la montre.

Selon une variante de l'invention, le bracelet de l'appareil selon l'invention est du type ouvrant, et comporte à chacune de ses extrémités, une électrode de prélèvement ; dans ce cas, il est possible d'effectuer un enregistrement au niveau du thorax en y appliquant ces deux électrodes.

Selon encore une autre variante de l'invention, la deuxième électrode est reliée à une électrode disposée sous un bracelet fixé sur le deuxième bras par l'intermédiaire d'un cordon de liaison. Dans ce cas, pour effectuer un enregistrement, il suffit de brancher cette électrode qui reste appliquée en permanence comme la première électrode.

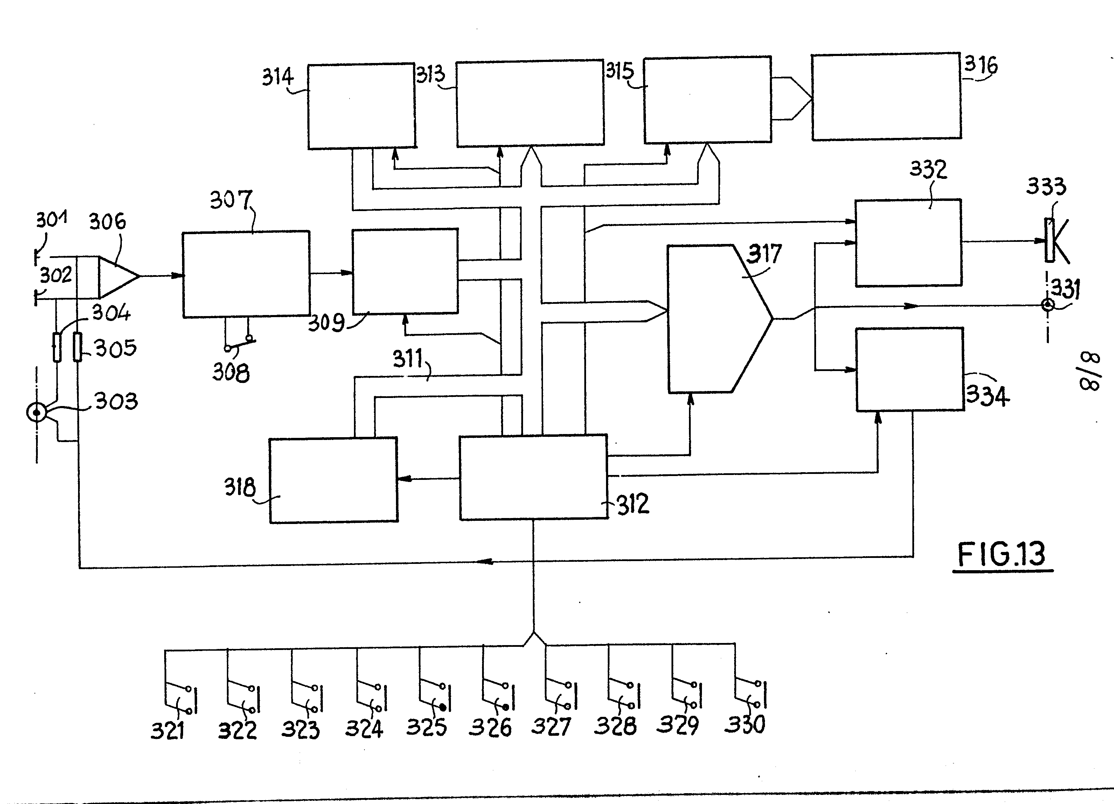

On reconnait sur la fig.13, les électrodes 301 et 302 qui sont fixées sur le boîtier ; conformément à l'invention, l'appareil comporte des électrodes extérieures, non représentées, qui sont reliées à l'appareil par un câble de liaison branché sur le connecteur 303. De ce fait, les deux électrodes extérieures sont branchées en parallèle sur les deux électrodes 301 et 302 du boîtier de l'appareil ; le branchement des éleectrodes extérieures sur les électrodes 301 et 302 du boîtier est réalisé par l'intermédiaire de filtres hautes fréquences 304 et 305 dont le rôle sera expliqué plus bas.

Un amplificateur différentiel 306 à haute impédance d'entrée reçoit le potentiel d'électrocardiogrammes venant des électrodes et le signal obtenu est envoyé sur un circuit 307 de recentrage automatique de trace qui réalise en particulier une compensation d'offset et qui comporte également deux constantes de temps commutables, ce qui permet la sélection de deux fréquences de coupure basse, par exemple RR = 0,5 Hz et ST = 0,05 Hz à -3 dB, la commutation s'effectuant au moyen d'un interrupteur 308.

On réalise également une atténuation de 12dB/octave des fréquences supérieures à 50 Hz au moyen d'un filtre actif passe-bas, ce qui a pour but d'éliminer les risques de recomposition de fréquence par battement avec le rythme d'échantillonnage qui est utilisé pour la conversion numérique du signal.

Le signal obtenu est envoyé sur un convertisseur analogique-numérique 309 qui est avantageusement un circuit à approximations successives intégré qui fournit un échantillon d'électrocardiogrammes codés sur un format de huit bits.

Le signal à huit bits obtenu est envoyé sur le bus 311 d'un microprocesseur 312 qui commande le fonctionnement de l'appareil. Ce bus est également relié à une mémoire vive (RAM), à une mémoire morte programmable (PROM) 314, à une mémoire vive 315 commandant un écran alphanumérique et graphique à cristaux liquides 316, à un circuit d'interpolateur linéaire 317 et à une horloge fonctionnant en temps réel 318.

Le microprocesseur 312 exécute, en fonction d'un programme enregistré dans la mémoire 314, les fonctions suivantes :

- génération d'images et écriture de caractères sur l'écran 316 ;

- écriture et lecture de la mémoire 315 dans laquelle est mémorisé le signal d'électrocardiogrammes et qui sert au rafraichissement de l'écran 316 ;

- commande des convertisseurs 319 et 317 ;

- calcul du rythme cardiaque par détection du segment QRS de chaque complexe de l'électrocardiogramme ;

- écriture et lecture de la mémoire 313 dans laquelle sont enregistrées la date et l'heure de l'enregistrement ;

- commande de l'horloge 318, et

- exploration et question des différentes touches de fonction de sélection 321 à 330.

Le circuit 317 est essentiellement constitué par un interpolateur linéaire et réalise la conversion des signaux à huit bits en un signal analogique qui est envoyé sur une prise 331 qui permet le branchement sur un appareil de lecture disposé chez le médecin traitant, sur un dispositif de transmission acoustique constitué par un oscillateur à fréquence commandé par la tension 332 dont le signal de sortie assure l'excitation d'un transducteur acoustique 333 constitué par exemple par un piézo-transducteur et à un dispositif de transmission par voie hertzienne 334 constitué essentiellement par un émetteur dont la sortie est reliée au câble de liaison des électrodes extérieures au boîtier ; les signaux haute fréquence sont bloqués par les filtres 304 et 305 afin d'éviter leur injection dans le circuit électronique de l'appareil. Le dispositif de transmission par voie hertzienne 334 émet à faible distance et ces signaux peuvent être reçu et décodés par un appareil récepteur disposé à proximité, ce qui permet, après avoir jugé de la qualité de l'enregistrement au moyen de l'écran, de commander aussitôt la lecture par le récepteur et la mise sous forme lisible par le médecin traitant. Avantageusement, l'émetteur 334 est un émetteur à modulation de fréquence.

Selon l'invention, l'appareil est en état de veille et effectue de manière continue l'enregistrement des électrocardiogrammes dans la mémoire 315, et lorsque l'on commande l'enregistrement en agissant sur la touche 328, on enregistre les signaux d'électrocardiogrammes qui se sont produits avant le déclenchement et après le déclenchement, par exemple sur une période de 10 secondes avant et de 10 secondes après le déclenchement. De cette manière, on est assuré d'avoir un enregistrement correspondant au début du malaise, ce qui peut aider au diagnostic. La touche 321 permet de sél"ctionner la durée de l'enregistrement, par exemple 20 ou 40 secondes. La touche 329 commande l'émission par voie hertzienne au moyen de l'émetteur 334.

L'actionnement de la touche 330 commande l'envoi de l'enregistrement effectué par le dispositif de transmission acoustique 332 et 333 dont le signal peut être envoyé sur un récepteur comportant un microphone et un détecteur comportant par exemple une boucle à verrouillage de phase et un interpolateur linéaire soit directement, soit par l'intermédiaire d'une ligne téléphonique.

Selon une caractéristique de l'invention, l'électrocardiogramme qui est prélevé pour les électrodes est transmis directement et de manière continue par le dispositif de transmission acoustique sans enregistrement dans la mémoire ; ceci permet d'effectuer une surveillance continue de l'état cardiaque d'un malade se trouvant dans un endroit isolé, par exemple un bateau.

La présence d'une horloge dans le circuit permet d'enregistrer l'heure et la date du signal dans la mémoire 313 et ces données sont portées sur l'enregistrement de l'électrocardiogramme qui est fourni au médecin traitant.

Selon l'invention, le microprocesseur 312 effectue de manière continue le calcul récurrent du rythme cardiaque par la détection du segment QRS et la valeur instantanée du rythme cardiaque est affichée sur l'écran 316 pendant toute la durée de l'enregistrement. Les fluctuations de cette valeur du rythme cardiaque permettent ainsi de donner une première indication sur la nature de la maladie.

La description ci-dessus n'a été fournie qu'à titre d'exemples non limitatifs, et il est évident que l'on peut y apporter des modifications ou variantes sans pour autant sortir du cadre de la présente invention.

Ainsi, comme indiqué plus haut, l'appareil peut être commandé au moyen d'un microprocesseur pouvant effectuer des traitements supplémentaires de l'électrocardiogramme détecté, tels que le calcul de la fréquence cardiaque, la reconnaissance des principales arythmies et leur comptage. Le résultat de ces calculs d'arythmies est alors transmis en même temps que le ou les électrocardiogrammes enregistrés.

Par ailleurs, on peut prévoir des mémoires de plus grande capacité afin d'augmenter la durée totale de l'enregistrement.

On peut aussi utiliser des mémoires à bandes magnétiques telles que des cassettes.

On peut également prévoir un dispositif indicateur du nombre de séquences qui ont déjà été effectuées ou qui restent à effectuer.

Enfin, on peut prévoir une touche accessible au patient et qui ne permette que la lecture de la mémoire sans effacement, en vue par exemple, d'une transmission téléphonique.

## REVENDICATIONS

1°) Appareil portatif d'enregistrement d'électrocardogrammes, caractérisé en ce qu'il comprend un boitier (1,101, 201) de faibles dimensions comportant, sur au moins une de ses faces extérieures (4), deux électrodes (5,6,102,103,202,204) d'enregistrement d'électrocardiogrammes destinées à recevoir chacune une partie d'un des deux bras et un dispositif électronique de traitement disposé dans ledit boitier et comprenant des circuits de numérisation (29,222,309), de mémorisation (31,226,313,315) et de conversion sous forme analogique (32, 225,317) du signal électrique détecté.

2°) Appareil selon la revendication 1, caractérisé en ce que le boitier est un boitier plat de format de poche (1) et en ce que les deux électrodes (5,6,102,103) sont disposées sur une même face (4) du boitier et reçoivent chacune un ou plusieurs doigts d'une main.

3°) Appareil selon la revendication 1, caractérisé en ce que le boitier est un boitier plat (201) du type boitier de montre-bracelet maintenu sur le bras au moyen d'un bracelet, en ce que la première électrode (202) est disposée sur la face du boitier qui est en contact avec le bras et en ce que la deuxième électrode (204) est disposée sur la face opposée extérieure du boitier (201) et destinée à recevoir une partie de l'autre bras telle qu'un doigt.

4°) Appareil selon la revendication 2, caractérisé en ce qu'il comporte des moyens de retenue (105,106,113,114) disposés sensiblement en regard des électrodes (102,103) et exerçant une pression sur la partie de la main en contact avec ces dernières de manière à réaliser un bon contact en évitant toute crispation.

5°) Appareil selon l'une des revendications précédentes, caractérisé ence qu'il comporte une touche de déclenchement de l'enregistrement (12,136) disposée sur l'extérieur du boitier (1,101) et actionnée par un doigt.

6°) Appareil selon la revendication 1, caractérisé en ce que le dispositif électronique de traitementcomprend un dispositif de transmission acoustique (228,229,332,333) émettant une onde sonore modulée par le signal d'électrocardiogrammes.

7°) Appareil selon la revendication 1, caractérisé en ce qu'il comporte un dispositif de transmission par voie hertzienne à faible distance (334) du signal d'électrocardiogrammes.

8°) Appareil selon la revendication 1, caractérisé en ce qu'il comporte une montre à affichage numérique (13,232, 318) incorporée et en ce que l'on enregistre l'heure et la date du prélèvement dans le circuit de mémorisation (31,226,313).

9°) Appareil selon la revendication 1, caractérisé en ce que le dispositif électronique de traitement comporte un dispositif (224,231,312) de calcul récurrent du rythme cardiaque et un dispositif d'affichage (206,316) affichant la valeur instantanée du rythme cardiaque pendant l'enregistrement.

10°) Appareil selon la revendication 5, caractérisé en ce qu'il comporte deux électrodes extérieures qui sont fixées en contact permanent avec le patient et reliées aux deux électrodes (301,302) du boitier et en ce que l'actionnement du dispositif de commande de l'enregistrement (328) dont le déclenchement commande l'enregistrement de l'électrocardiogramme pendant une période s'écoulant avant et après ledit déclenchement.

11°) Appareil selon la revendication 9, caractérisé en ce que le dispositif électronique de traitement comprend des détecteurs de tachycardie et de brachycardie à seuils réglables.

12°) Appareil selon la revendication 6, caractérisé en ce que le signal d'électrocardiogrammes est transmis de manière continue par le dispositif de transmission acoustique sans enregistrement dans les circuits de mémorisation.

**FIG.1**

0106756

# FIG.2

5   6

2   - 4 -   3

# FIG.3

23-43   22-42

28   31

5

6

26   27   36   29

12   ⊗

14   21-41   32   24-44   25-45

33   34

# FIG.4

## FIG.5

## FIG.6

## FIG.7

FIG.8

FIG.9

0106756

FIG.10

FIG.11

FIG.12

7/8

0106756

FIG.13

8/8

0106756

**0106756**

Office européen
des brevets

**RAPPORT DE RECHERCHE EUROPEENNE**

Numéro de la demande

EP    83 40 1949

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| P,X | FR-A-2 519 856  (MICRO-MED)<br><br>* En entier * | 1,2,5, 10 | A 61 B    5/04 |
| | --- | | |
| A | FR-A-2 401 647  (A.E. KARZ)<br><br>* Page 6, ligne 25 - page 22, ligne 24; figures 1-10 * | 1,5-7, 11,12 | |
| | --- | | |
| A | DE-A-2 604 460  (M.S. LAMPADIUS)<br><br>* Page 15, ligne 1 - page 19, ligne 17; figures 1-5 * | 1,8,10 ,11 | |
| | --- | | |
| A | DE-A-2 738 605  (D.L. WOLFE et al.)<br>* Page 8, ligne 21 - page 12, ligne 7; figures 1,4 * | 3,5,8, 9 | |
| | --- | | **DOMAINES TECHNIQUES RECHERCHES (Int. Cl. ³)** |
| A | US-A-3 732 868  (L.F. WILLEMS et al.)<br>* En entier * | 1,2,6 | A 61 B    5/04 |
| | --- | | |
| A | FR-A-2 361 857  (MEDTRONIC INC.)<br>* Page 1, ligne 1 - page 5, ligne 19; figures 1-3 * | 2,3,8 | |
| | --- | | |
| A | US-A-3 885 552  (J.R. KENNEDY)<br>* Abrégé; colonne 9, ligne 30 - colonne 10, ligne 63; figures 1a,1b * | 4,6,12 | |
| | ---                    -/- | | |

Le présent rapport de recherche a été établi pour toutes les revendications

| Lieu de la recherche<br>LA HAYE | Date d'achèvement de la recherche<br>06-12-1983 | Examinateur<br>ZILLIOX J.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

Office européen
des brevets

**RAPPORT DE RECHERCHE EUROPEENNE**

Numéro de la demande

EP 83 40 1949

| | **DOCUMENTS CONSIDERES COMME PERTINENTS** | | **Page 2** | |
|---|---|---|---|---|
| Catégorie | Citation du document avec indication, en cas de besoin, des parties pertinentes | Revendication concernée | CLASSEMENT DE LA DEMANDE (Int. Cl. ³) | |
| A | US-A-3 841 314 (R.E. PAGE)<br>* Colonne 4, ligne 61 - colonne 5, ligne 50; figure 4 *<br><br>--- | 4 | | |
| A | US-A-3 960 140 (R.L. BUXTON)<br>* Colonne 3, ligne 10 - colonne 4, ligne 21; figure 1 *<br><br>--- | 7 | | |
| A | DE-A-2 839 331 (A. AMMERSCHLÄGER et al.)<br>* Page 10, ligne 6 - page 15, ligne 11; figures 1-4 *<br><br>--- | 1,5,10 | | |
| A | DE-A-2 603 628 (LRE RELAIS & ELEKTRONIK GmbH)<br>* Page 8, lignes 1-4; figure 2 *<br><br>----- | 2 | | |
| | | | **DOMAINES TECHNIQUES RECHERCHES (Int. Cl. ³)** | |

Le présent rapport de recherche a été établi pour toutes les revendications

| Lieu de la recherche<br>LA HAYE | Date d'achèvement de la recherche<br>06-12-1983 | Examinateur<br>ZILLIOX J.M. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82